# EUROPEAN PATENT SPECIFICATION

(11) **EP 3 447 137 B1**
(45) Date of publication and mention of the grant of the patent: **26.08.2020**
(21) Application number: 16890284.9
(22) Date of filing: 16.05.2016
(51) Int. Cl.: C12N 9/96, C12N 9/10

(54) **LIQUID ENZYME PREPARATION AND PREPARATION METHOD THEREOF**
FLÜSSIGE ENZYMZUBEREITUNG UND HERSTELLUNGSVERFAHREN DAFÜR
PRÉPARATION D'ENZYME LIQUIDE ET SON PROCÉDÉ DE PRÉPARATION

(30) Priority: 16.02.2016 CN 201610086526
(43) Date of publication of application: 27.02.2019
(73) Proprietor: Kinry Food Ingredients Co., Ltd., Shanghai 201802 (CN); Kinry Biotech (Jinan) Co., Ltd., Jinan, Shandong 251606 (CN)
(72) Inventor: WANG, Fangming, Shanghai 201802 (CN); XIE, Yongqiang, Shanghai 201802 (CN); XU, Baodi, Shanghai 201802 (CN)
(74) Representative: dompatent von Kreisler Selting Werner - Partnerschaft von Patent- und Rechtsanwälten mbB
(86) International application number: PCT/CN2016/082178
(87) International publication number: WO 2017/140051

(56) References cited:
- EP-A1- 2 251 421
- CN-A- 104 024 406
- CN-A- 105 462 950
- JEITNER T M ET AL: "Mechanism for the inhibition of transglutaminase 2 by cystamine", BIOCHEMICAL PHARMACOLOGY, ELSEVIER, US, vol. 69, no. 6, 15 March 2005 (2005-03-15) , pages 961-970, XP027715596, ISSN: 0006-2952 [retrieved on 2005-03-15]
- KIELISZEK MAREK ET AL: "Microbial transglutaminase and its application in the food industry. A review", FOLIA MICROBIOLOGICA, SPRINGER NETHERLANDS, NL, vol. 59, no. 3, 8 November 2013 (2013-11-08), pages 241-250, XP035323972, ISSN: 0015-5632, DOI: 10.1007/S12223-013-0287-X [retrieved on 2013-11-08]
- WANG, MEILING: "Microbial Trans-glutaminase and Its Application in Food Industry", SHANDONG FOOD FERMENT, 28 February 2015 (2015-02-28), pages 34-36, XP009512226, ISSN: 1671-5799
- ZHAO, HUAMEI et al.: "Study on Trans-glutiminase in Food Industry", FOOD SCIENCE AND TECHNOLOGY, no. 9, 31 December 2008 (2008-12-31), pages 137-141, XP009511564, ISSN: 0022-1155

## Description

### Technical Field

The invention discloses a liquid enzyme preparation and a method for preparing the same, in particular, relates to a liquid preparation of transglutaminase (EC2.3.2.13) and a preparation method thereof, wherein the liquid preparation can be stored at room temperature, being stable in property, with the enzyme activity maintained at a level of above 80%. The invention belongs to the field of enzyme preparations and the field of food additives.

### Background

Transglutaminase (EC.2.3.2.13, full name: Amine γ-glutamyl-transferase, abbreviated as TG) is a cross-linking protein enzyme that can catalyze the formation of an ε- (γ-glutamyl) lysine isopeptide bond in a protein molecule or molecules between proteins by using an ε-amino group of lysine in the peptide chain of a protein as Acyl acceptor, improve the properties of the protein such as emulsibility, rheological characteristic, solubility and foamability, confer protein-specific texture characteristics and adhesivity, and improve the color, flavor and taste of products, thus have a great commercial value.

Transglutaminase was first isolated from liver of guinea pig by Clarke *et al.* In 1989, Ando *et al.* from Amano Pharmaceutical Co., Ltd. (Japan) invented a microbial fermentation process for the production of transglutaminase. In 1997, Ajinomoto Co., Inc. (Japan) accomplished commercial-scale production of transglutaminase. In 2001, transglutaminase was officially approved as food additive in China. Currently, the primary fermentation manufacturer in the world is Ajinomoto Co., Inc. (Japan), whose transglutaminase product has a brand name of Acvita. The primary manufacturers in China are Shanghai Kinry Food Ingredients Co., Ltd., Kinry Biotech (Jinan) Co., Ltd., Taixing Dongsheng Bio-Tech Co., Ltd., Taixing Yiming Biological Product Co. Ltd., Henan Yangshao Bio-products Co., Ltd., etc. Their products are enzyme preparations in a solid powder form, with Shanghai Kinry Food Ingredients Co., Ltd. being the first to use vacuum packaging. Ajinomoto Co., Inc. (Japan) currently employs nitrogen-filled packaging for powder products.

Although powder preparations of transglutaminase have been produced in a large scale and are widely applied in the food processing industry now, there are still a lot of problems in their production, transportation, storage and application.
1) Production cost: during the production of transglutaminase, a fermentation liquid is obtained first, which requires several processing steps during its refinement, such as precipitation, drying, and grinding; due to the complicated processes, the production cost is increased.
2) Safety protection: since powder preparations of transglutaminase are easy to disperse during production and use, workshop environment gets worse, and the equipments are difficult to clean. Working place is full of dust, which is difficult to be controlled and protected. Once enzyme powder is inhaled into human bodies, it causes a strong anaphylactic response and seriously hurt the organs of respiratory tract in operators, who may have symptoms such as high fever, pneumonia and shock, and therefore it is quite harmful to the health of operators. So far, events of allergy symptoms caused by transglutaminase, such as cough and fever, have occurred in factories for many times.
3) Packaging problems: vacuum packaging is used for powder enzyme preparations of transglutaminase. However, the powder easily enters the equipment when vacuumizing, resulting in considerable loss. If the enzyme preparation package is not sealed tightly and air leakage occurs, it will be humidified, agglomerated, with the enzyme activity reduced. If the nitrogen-filled packaging is used, the nitrogen-filling operation is tedious and leads to dust raising, and the production cost is increased greatly.
4) With respect to application: it is well known that transglutaminase can only work when being dissolved in water. Due to the secondary dissolution of a powder preparation during its production and application, the duration for the enzyme to work is greatly reduced. Moreover, incomplete dissolution often appears after the addition of water, while the direct addition of powder easily leads to uneven distribution. In addition, when a device is used to carry out the rolling and rubbing operation, the powder easily adheres to the device, reducing the utilization rate of the powder preparations, and negatively affecting the application. Therefore, the amount of the transglutaminase powder preparation has to be increased.
5) Development trend of enzyme preparations: in the field of biological enzyme preparations and various industrial fields, various kinds of enzyme preparations have been already available in liquid dosage forms, with the problems in storage, transportation and application having been solved, and the production and application thereof have been accomplished economically and environmentally, for example, liquid cellulase (Patent Publication No: CN103981168A, CN101381716), liquid phytase (Patent Publication No: CN101617740), liquid pectinase (Patent Publication No: CN104531653A), liquid lipase (Patent Publication No: CN103525797A). However, throughout the world, liquid preparations of transglutaminase, which can be stably stored at room temperature for a long time, are not commercially available yet.

European Patent Application EP2251421 discloses the use of stabilised transgluatminase solutions that may contain several groups of stabilisers such as trehalose, sucrose, soybean or wheat or milk extracts, and antioxidants in general, and which may further contain specific salts, such as citrate, carbonate or ascorbate.

Patent Publication No: CN104024406A provides a preparation method for a liquid enzymatic preparation of transglutaminase. However, since the method only achieves the storage at a low temperature for up to 180 days, and the storage and transportation of the product have to be performed under freezing conditions, the commercialized use of the method has been limited, and there are no final products of the method yet. How to develop a liquid enzyme preparation of transglutaminase, which is stable at room temperature (25°C, the same below), has now become a problem which needs to be solved urgently in food industry now.

### Description of the Invention

One object of the invention is to provide a liquid preparation of transglutaminase (EC2.3.2.13), which can be stored at room temperature for 6 months and maintains the enzyme activity of above 80%.

Another object of the invention is to provide a method for preparing a liquid preparation of transglutaminase (EC2.3.2.13), wherein the liquid preparation of transglutaminase prepared by the method has good stability, can be stored at room temperature for 6 months, and maintains the enzyme activity of above 80%.

The invention primarily solves the technical problem concerning the difficulty in storage of liquid preparations of transglutaminase at room temperature, and the liquid preparations of transglutaminase can meet the requirements in commercial production and application.

In order to achieve the above objects, the inventive technical solutions need to be illustrated experimentally and theoretically first, and then summarized. Details are as follows.

Firstly, by investigating the effects of physicochemical factors such as pH, water activity, and redox potential on the preservation rate of enzyme activity (stability) of a transglutaminase solution at room temperature, the optimal pH range, water activity, and redox potential range for the stable storage of transglutaminase at room temperature were determined.

### 1. Stability of transglutaminase solutions at different pH values

To concentrated enzyme solutions, hydrochloric acid and sodium hydroxide were added for preparing transglutaminase solutions at different pH values. After sterile filtration, the transglutaminase solutions were kept at room temperature for 2 days to evaluate their enzyme activity (stability). The results were shown in Table 1.

**Table 1. Changes in the enzyme activities of purified concentrated enzyme solutions at different pH**

| **pH regulator** | **pH** | **Enzyme activity after adjustment** (µ/ml) | **Enzyme activity 2 days later** (µ/ml) | **Preservation rate %** |
|---|---|---|---|---|
| HCL | 4.0 | 85.6 | 60.5 | 70,68% |
| HCL | 5.0 | 95.2 | 90.3 | 94.85% |
| NaOH | 6.0 | 99.4 | 98.6 | 99.20% |
| NaOH | 7.0 | 99.6 | 98 5 | 98.90% |
| NaOH | 7.5 | 99.2 | 93.5 | 93.80% |
| NaOH | 8.0 | 98.9 | 89.1 | 90.10% |
| NaOH | 9.0 | 95.5 | 78.1 | 81.78% |
| NaOH | 10.0 | 90.2 | 50.4 | 55.88% |

The data in Table 1 showed that, when transglutaminase was kept at a pH of 5.0-9.0, the purified enzyme solutions had a preservation rate of enzyme activity of above 80%, while when transglutaminase was kept at a pH of above 9.0 or below 4.0, the enzyme activity reduced quickly. Therefore, the enzyme solution could be stored at a pH of 5.0-9.0, and the preferred pH is 5.0-7.5.

### 2. Stability of transglutaminase solutions at different water activities

A pH regulator was used to adjust the pH of a purified enzyme solution to 6.0 first, and the enzyme solution was diluted with a regulating buffer to 1000 u/ml. To 10ml diluted enzyme solution, glycerol was added in different amounts (Table 2), and the resulting solutions were diluted with buffer to a volume of 100ml. After sterile filtration, liquid enzyme preparations with different water activities were obtained. After being kept at room temperature for 10 days, the stability of the liquid enzyme preparations was evaluated, and the results were shown in Table 2.

**Table 2. The enzyme activities (stability) of purified concentrated enzyme solutions at different water activities**

| **Liquid enzyme(ml)** | **Glycerol (g)** | **Aw** | **Enzyme activity after adjustment(u/ml)** | **Enzyme activity 10 days later (u/ml)** | **Preservation rate %** |
|---|---|---|---|---|---|
| 10 | 0 | 0.99 | 1 00 | 57.11 | 57.10 |
| 10 | 10 | 0.95 | 100 | 69.96 | 69.96 |
| 10 | 20 | 0.92 | 100 | 75.25 | 75.25 |
| 10 | 30 | 0.89 | 100 | 85.92 | 85.92 |
| 10 | 40 | 0.85 | 100 | 92.19 | 92.19 |
| 10 | 50 | 0.80 | 100 | 96.61 | 96.61 |
| 10 | 60 | 0.75 | 100 | 9601 | 96.01 |
| 10 | 70 | 0.62 | 100 | 96.69 | 96.69 |
| 10 | 80 | 0.52 | 100 | 98.54 | 98.54 |
| 10 | 90 | 0.31 | 100 | 98.10 | 98.10 |

The data in Table 2 showed that the addition of a water activity regulator could enhance the stability of the liquid preparation of transglutaminase at room temperature. When the water activity was lower than 0.89, the preservation rate of enzyme activity reached above 85% at Day 10, and when the water activity was lower than 0.85, the preservation rate of enzyme activity reached 92%. However, a further decrease in water activity had little effect on the preservation rate of enzyme activity. Therefore, a good preservation rate could be obtained when the water activity was lower than 0.89. However, in view of the cost of raw material, the preferred water activity was in the range of 0.60-0.85.

### 3. Stability of transglutaminase solutions at room temperature under different redox potential conditions

A pH regulator was added to adjust the pH of a purified concentrated enzyme solution to 6.0. And then, the redox potentials of the enzyme solution were adjusted to different values with the redox potential regulators of sodium borohydride and hydrogen peroxide. After sterile filtration, the enzyme solution was kept at room temperature for 30, 60, and 90 days, and the enzyme activities and the preservation rates of the sample enzyme solutions were determined. The results were shown in Table 3.

**Table 3. The enzyme activities and preservation rates of liquid preparations of transglutaminase at different redox potentials**

| **Potential** | **Initial (u/ml)** | **30 d** | | **60 d** | | **90 d** | |
|---|---|---|---|---|---|---|---|
| | | **Enzyme activity (U/ml)** | **Preservation rate (%)** | **Enzyme activity (U/ml)** | **Preservation rate (%)** | **Enzyme activity (U/ml)** | **Preservation rate (%)** |
| | | | | | | | |
| -700mv | 98.3 | 49.81 | 50.67% | 39.84 | 40.53% | 29.9 | 30.40% |
| -600mv | 99.1 | 76.64 | 77.33% | 61.31 | 61.87% | 46 | 46.40% |
| -400mv | 99.4 | 94.30 | 94.87% | 92.14 | 92.69% | 86.4 | 86.90% |
| -200mv | 99.4 | 95.21 | 95.78% | 92.80 | 93.36% | 87 | 87.53% |
| -150mv | 99.1 | 95.32 | 96.19% | 94.39 | 95.24% | 88.5 | 89.29% |
| -100mv | 99.4 | 96.41 | 96.98% | 94.89 | 95.47% | 89 | 89.50% |
| -50mv | 99.3 | 94.83 | 95.50% | 94,69 | 95.36% | 88.8 | 89.40% |
| 0mv | 99.1 | 93.42 | 94.27% | 85.41 | 86.19% | 80.1 | 80.80% |
| 50mv | 98.7 | 81.05 | 82.12% | 74,11 | 75.08% | 69.5 | 70.39% |
| 100mv | 98.4 | 24.30 | 24.70% | 19.44 | 19.76% | 14.6 | 14.82% |
| 150mv | 98.6 | 9.24 | 9.37% | 7.39 | 7.49% | 5.5 | 5.62% |
| 200mv | 98.5 | 7.70 | 7.82% | 6.16 | 6.25% | 4.6 | 4.69% |

The data in Table 3 showed that the redox potential had a great effect on the stability of a liquid preparation of transglutaminase. When the redox potential was in a range of -400 mv to 50 mv, the preservation rate was more than 80% at room temperature after 30 days. When the redox potential of a liquid enzyme preparation was lower than 50 mv, the preservation rate of enzyme activity was increased greatly. When the potential was between -400 mv and 0mv, the preservation rate would reach above 80% after 90 days. The preferred potential for the liquid enzyme preparation was between -400 mv and 0 mv.

Secondly, pH regulators, water activity regulators, and redox potential regulators were evaluated in terms of their effects on the stability of a liquid transglutaminase, thereby selecting suitable pH regulators, water activity regulators, and redox potential regulators.

### 4. Effect of different pH regulators on stability of transglutaminase solutions

One of hydrochloric acid, sulfuric acid, acetic acid, lactic acid, citric acid, malic acid, phytic acid, phosphoric acid, nitric acid, oxalic acid, sodium hydroxide, potassium hydroxide, sodium carbonate, sodium bicarbonate, potassium carbonate, potassium bicarbonate, trisodium citrate, tripotassium citrate, sodium acetate, potassium acetate, sodium lactate, potassium lactate, disodium hydrogen phosphate, dipotassium hydrogen phosphate, sodium phosphate, potassium phosphate, tap water, purified water, and mineral water, or a buffer system consisting of several pH regulators was selected to adjust the pH of a purified transglutaminase solution to the range as listed in Table 1. Different pH regulators and random combinations of different pH regulators were investigated for their effect on the stability of a liquid transglutaminase at room temperature. The results were shown in Tables 4-1, 4-2, and 4-3.

**Table 4-1. Effects of different alkaline pH regulators on stability of a liquid transglutaminase**

| **Type** | **pH** | **Enzyme activity after adjustment (u/ml)** | **Enzyme activity 2 days later (u/ml)** | **Preservation rate (%)** |
|---|---|---|---|---|
| Sodium hydroxide | 6.0 | 99.0 | 98.5 | 99.49 |
| Potassium hydroxide | 6.0 | 99.6 | 98.7 | 99.10 |
| Sodium carbonate | 6.0 | 99.7 | 98.4 | 98.70 |
| Sodium bicarbonate | 6.0 | 99.5 | 98.1 | 98.59 |
| Potassium carbonate | 6.0 | 99.7 | 98.4 | 98.70 |
| Potassium bicarbonate | 6.0 | 99.1 | 98.1 | 98.99 |
| Trisodium citrate | 6.0 | 98.9 | 98.0 | 99.09 |
| Tripotassium citrate | 6.0 | 99.5 | 99.1 | 99.60 |
| Sodium acetate | 6.0 | 99.6 | 98.6 | 99.00 |
| Potassium acetate | 6.0 | 99.7 | 98.5 | 98.80 |
| Sodium lactate | 6.0 | 99.4 | 98.7 | 99.30 |
| Potassium lactate | 6.0 | 99.3 | 98.4 | 99.09 |
| Disodium hydrogen phosphate | 6.0 | 99.8 | 98.7 | 98.90 |
| Dipotassium hydrogen phosphate | 6.0 | 99.2 | 98.0 | 98.79 |
| Sodium phosphate | 6.0 | 99.5 | 98.4 | 98.89 |
| Potassium phosphate | 6.0 | 99.9 | 98.8 | 98.90 |
| NaOH+KOH (1:1) | 6.0 | 99.8 | 98.5 | 98.70 |
| NaOH+KOH+Na₂CO₃ (1:1:1) | 6.0 | 99.1 | 98.4 | 99.29 |
| Trisodium citrate + Sodium carbonate (0.1:0.9) | 6.0 | 99.6 | 98.2 | 98.59 |
| Sodium phosphate + Potassium lactate (0.2:0.8) | 6.0 | 98,9 | 98.4 | 99.49 |

**Table 4-2. Effects of different acidic pH regulators on the stability of a liquid transglutaminase**

| **Type** | **pH** | **Enzyme activity after adjustment (u/ml)** | **Enzyme activity 2 days later (u/ml)** | **Preservation rate (%)** |
|---|---|---|---|---|
| HCl | 5.5 | 99.1 | 98.4 | 99.29 |
| H₂SO₄ | 5-5 | 99.6 | 98.6 | 99.00 |
| Acetic acid | 55 | 99.7 | 98.4 | 98.70 |
| Lactic acid | 55 | 99.7 | 98.7 | 99.00 |
| Citric acid | 5.5 | 99.5 | 98.6 | 99.10 |
| Malic acid | 5.5 | 99.1 | 98.5 | 99.39 |
| Phytic acid | 5.5 | 99.4 | 99.0 | 99.60 |
| Phosphoric acid | 5.5 | 99.5 | 98.8 | 99.30 |
| Nitric acid | 55 | 99.8 | 98.9 | 99.10 |
| Oxalic acid | 5.5 | 99.2 | 98.7 | 99.50 |
| HCl+H₂SO₄ (1:1) | 5.5 | 993 | 98.3 | 98.99 |
| HCl+Lactic acid (1:1) | 5.5 | 99.4 | 98.4 | 98.99 |
| Acetic acid+Citric acid+H₂SO₄ (1:1:1) | 5.5 | 99.0 | 98.1 | 99.09 |

**Table 4-3. Effects of different combinations of pH regulators on the stability of a liquid transglutaminase**

| **Type** | **pH** | **Enzyme activity after adjustment (u/ml)** | **Enzyme activity 2 d later (u/ml)** | **Preservation rate (%)** |
|---|---|---|---|---|
| purified water | 5.8 | 99.1 | 98.8 | 99.70 |
| tap water | 6.7 | 99.5 | 98.8 | 99.30 |
| mineral water | 5.9 | 99.7 | 98.4 | 98.70 |
| 0.02M sodium phosphate buffer | 7.0 | 99.8 | 98.1 | 98.0 |
| 0.02M sodium citrate buffer | 6.0 | 99.5 | 98.4 | 98.89 |
| 0.02M sodium acetate buffer | 5.5 | 99.6 | 99.1 | 99.50 |
| 0.02M sodium lactate buffer | 5.5 | 99.3 | 98.4 | 99.09 |

The data in Tables 4-1, 4-2, and 4-3 showed that there were no significant differences and changes regarding the effects of the selected acidic pH regulators, alkaline pH regulators, and the like on the storage of the enzyme solution at room temperature. Therefore, any of hydrochloric acid, sulfuric acid, acetic acid, lactic acid, citric acid, malic acid, phytic acid, phosphoric acid, nitric acid, oxalic acid, sodium hydroxide, potassium hydroxide, sodium carbonate, sodium bicarbonate, potassium carbonate, potassium bicarbonate, trisodium citrate, tripotassium citrate, sodium acetate, potassium acetate, sodium lactate, potassium lactate, disodium hydrogen phosphate, dipotassium hydrogen phosphate, sodium phosphate, potassium phosphate, tap water, purified water, and mineral water, or a buffer system consisting of several pH regulators of the above could be selected as the pH regulator to adjust the pH of a transglutaminase liquid. In view of the safety in production operations and the effects of the transiently extreme pH of a strong acid or a strong base on the activity of transglutaminase during the adjustment operation, a buffer comprising phosphoric acid, acetic acid, lactic acid or citric acid or a salt thereof, is preferred.

### 5. Effects of different water activity regulators and amounts thereof on the stability of liquid transglutaminase at room temperature

The effects of different water activity regulators on the stability of transglutaminase solutions was tested as followed: under room temperature and sterile conditions, to a transglutaminase solution, one or more of sorbitol, maltitol, propylene glycol, glycerol, xylitol, polyethylene glycol (PEG) 400, PEG 600, PEG 800, PEG 20000, glucose, trehalose, sucrose, maltose, isomaltose, maltodextrin, and xylitol were added, to adjust the water activity of the liquid enzyme preparation to 0.75, and the liquid enzyme preparation was kept under a sterile condition for 10 days. The results were shown in Table 5-1.

**Table 5-1. Effects of different water activity regulators on the enzyme activity of transglutaminase**

| **Type** | **Initial enzyme activity (u/ml)** | **Water activity** | **Preservation rate of enzyme activity (%)** |
|---|---|---|---|
| Sorbitol | 99.4 | 0.75 | 81.89% |
| Maltitol | 98.5 | 0.75 | 83.76% |
| Propylene glycol | 98.4 | 0.75 | 84.55% |
| Glycerol | 97.5 | 0.75 | 95.44% |
| Trehalose | 98.6 | 0.75 | 83.77% |
| PEG 400 | 97 5 | 0.75 | 86.36% |
| PEG 600 | 98.6 | 0.75 | 84.79% |
| PEG 800 | 98.4 | 0.75 | 83.43% |
| PEG 20000 | 98.7 | 0.75 | 84.90% |
| Glucose | 98.8 | 0,75 | 6.8.48% |
| Sucrose | 99.0 | 0.75 | 82.12% |
| Maltose | 99.1 | 0.75 | 81.23% |
| Isomaltose | 99.5 | 0.75 | 81.11% |
| Maltodextrin | 98.9 | 0.75 | 82.20% |
| Xylitol | 99.3 | 0.75 | 83.48% |
| Glycerol: Sorbitol(9 : 1) | 98.1 | 0.75 | 88.07% |
| Glycerol: Maltitol (9:1) | 97.6 | 0.75 | 88.22% |
| Glycerol: Maltitol (7:3) | 98.6 | 0.75 | 88.03% |
| Glycerol:PEG 600(9:1) | 97.1 1 | 0.75 | 89.29% |
| Maltitol: Sorbitol(9:1) | 98.4 | 0.75 | 88.52% |
| Propylene glycol: Glycerol: Maltitol (1:5:4) | 99.1 | 0.75 | 89.40% |
| Control | - | 0.99 | 39.31% |

The data in Table 5-1 showed that, when one or more of sorbitol, maltitol, propylene glycol, glycerol, xylitol, PEG 400, PEG 600, PEG 800, PEG 20000, trehalose, sucrose, maltose, isomaltose, maltodextrin, and xylitol were used as a water activity regulator to adjust the water activity of the liquid transglutaminase to 0.75, they had a good protective effect on the stability of the liquid enzyme preparation at room temperature. As shown in the results, sorbitol, maltitol, glycerol or any combination thereof was preferred. It can be used in an amount of 30-80% (w/v), preferably 30-70% (w/v).

Several water activity regulators in Table 5-1 were tested for the relationship between the amount used and the preservation rate of enzyme activity at room temperature. The liquid enzyme preparation was kept under a sterile condition at pH 6.0, then the preservation rates of enzyme activity (%) at room temperature were determined 10 days later. The results were shown in Table 5-2.

**Table 5-2. Effects of different water activity regulators on the enzyme activity of transglutaminase**

| **Regulator** | 20% (w/v) | 30% ( w/v ) | 40% (w/v) | 50% (w/v) | 60% (w/v) | 70% (w/v) | 80% (w/v) |
|---|---|---|---|---|---|---|---|
| A | 73.2 | 81.2 | 86.5 | 89.6 | 92.4 | 94.4 | 95.0 |
| B | 75.5 | 86.0 | 92.1 | 96.5 | 96.6 | 96.7 | 97.9 |
| C | 60.1 | 82.3 | 88.7 | 91.5 | 93.6 | 94.3 | 95.2 |
| D | 70.2 | 84.7 | 90.2 | 93.5 | 93.8 | 94.0 | 94.3 |
| E | 69.8 | 87.2 | 93.1 | 95.3 | 95.5 | 95.3 | 96.1 |
| F | 62.3 | 85.5 | 91.7 | 97.3 | 97.8 | 98.2 | 98.4 |
| G | 65.5 | 82.6 | 87.9 | 94.6 | 95.5 | 95.9 | 96.5 |
| H | 61.8 | 86.8 | 92.6 | 97.0 | 97.6 | 97.8 | 98.0 |

| | | | | | | | |
|---|---|---|---|---|---|---|---|
| Note: in Table 5-2, A represents maltitol; B represents glycerol; C represents trehalose; D represents PEG 600; E represents glycerol: sorbitol (9: 1); F represents glycerol: maltitol (7: 3); G represents maltitol: sorbitol (9: 1); and H represents propylene glycol: glycerol: maltitol (1: 5: 4). | | | | | | | |

The data in Table 5-2 showed that at an optimal pH, when the water activity regulator was used in an amount of above 30%, the above water activity regulators alone or in combination resulted in a preservation rate of enzyme activity of above 80% at room temperature 10 days later for the liquid enzyme preparation. When the water activity regulator was used in an amount of above 40%, the preservation rate of enzyme activity was above 86%. With the increase in the amount of water activity regulator(s), the preservation rate of enzyme activity increased, but not to a large extent. In view of the production cost, the amount of water activity regulator(s) is preferably between 30 and 70% (w/v).

### 6. Effects of different redox potential regulators on the stability of a liquid transglutaminase at room temperature

Within the preferred redox potential range, the inventors investigated the effects of different redox potential regulators on the stability of a liquid transglutaminase, with the redox potential regulator used in a maximum amount as specified in GB2760-2014 Standard. In the experiment, a citrate buffer (pH 6.0) was used to adjust the pH of a transglutaminase solution to 6.0, and glycerol was added to adjust the water activity to 0.73-0.75.

The data in Table 6 and Table 7 showed that, when the different redox potential regulators were used to adjust the redox potentials of liquid transglutaminase to between 50mv and -150mv; the preservation rate of enzyme activity the preparation with the addition of redox potential regulators reached above 80% after storage for 90 days at room temperature, indicating that the preparation was of commercial value. Except for some redox potential regulators such as sodium lactate and calcium lactate, all the other formulas comprising the redox potential regulators had a preservation rate of enzyme activity of above 80% after storage for 180 days at room temperature.

**Table 6. Effects of different redox potential regulators on the stability of liquid preparations of transglutaminase**

| **Potential regulator** | **Added amount (%)** | **Potential (mv)** | **Aw** | Initial activity (u/ml) | **30 d** | | **90 d** | | **180 d** | |
|---|---|---|---|---|---|---|---|---|---|---|
| | | | | | Activity | Preservation % | Activity | Preservation % | Activity | Preservation % |
| Phytic acid | 0.020% | -7 | 0.75 | 99.2 | 94.2 | 95.00% | 87.8 | 88.50% | 79.86 | 80.5% |
| Disodium edetate | 0.0030% | -3 | 0.75 | 99.3 | 94.1 | 94.80%_{.} | 87.4 | 88.00% | 80.43 | 81.0% |
| Calcium disodium edetate | 0.0075% | -3 | 0.75 | 99.4 | 94. 4 | 95. | 88.5 | 89.00% | 81.51 | 82.0% |
| Reduced glutathione | 0.10% | -40 | 0.74 | 100.0 | 95.5 | 93.50% | 93.0 | 93.00% | 88.00 | 88.0% |
| L-cysteine | 0.10% | -146 | 0.74 | 100.1 | 96.1 | 96.00% | 92.1 | 92.00% | 84.59 | 86.5% |
| L-serine | 0.10% | 0 | 0.74 | 99.4 | 93.4 | 94.00% | 89.5 | 90.00% | 84.49 | 85.0% |
| L-cysteine HCl | 0.12% | -130 | 0.74 | 99.6 | 95.6 | 96.00% | 89.9 | 90.30% | 84.16 | 84.5% |
| Ascorbic acid | 0.50% | -7 | 0.74 | 99.3 | 94.3 | 95,00% | 88.4 | 89.00% | 83.41% | 84.0% |
| Calcium ascorbate | 0.50% | -60 | 0.73 | 99.3 | 94.8 | 95.50% | 90.9 | 91.50% | 83.91 | 84.5% |
| Sodium ascorbate | 0.50% | -40 | 0,74 | 99.5 | 95.0 | 95.50% | 91.5 | 92.00% | 89.57 | 86.0% |
| D-isoascorbic acid | 0.50% | 4 | 0.74 | 99.5 | 94.5 | 95.00% | 91.0 | 91.50% | 85.07 | 85. 5% |
| Sodium D-isoascorbate | 0.50% | -42 | 0.74 | 99.5 | 95.0 | 95.50% | 89.6 | 90.00% | 84.38 | 84.8% |
| Sodium bisulfite | 0.02% | 5 | 0.75 | 99.3 | 89.4 | 90.00% | 85.4 | 86.00% | 79.94 | 80.5% |
| Sodium metabisulfite | 0.02% | 5 | 0.75 | 99.4 | 91.4 | 92.00% | 86.5 | 87.00% | 81.51 | 82.0% |
| Potassium metabisulfite | 0.02% | 5 | 0.75 | 99.4 | 90,5 | 91.00% | 86.5 | 87.00% | 81.51 | 82.0% |
| Sodium sulfite | 0.02% | -80 | 0.75 | 99.5 | 94.5 | 95.00% | 90.5 | 91.00% | 82.59 | 83.0% |
| sodium hyposuifite | 0.02% | -50 | 0.75 | 99.5 | 94.3 3 | 94.80% | 89.66 | 90.00% | 83.58 | 84.0% |
| Soybean protein hydrolysate | 1% | -10 | 0.73 | 99.4 | 95.4 | 96.00% | 91.4 | 92.00% | 85.48 | 86.0% |
| Wheat protein hydrolysate | 1% | -30 | 0.73 | 99.1 | 94.6 | 95. | 90.98 | 91.60% | 84.73 | 85.5% |
| Sodium caseinate | 1% | -30 | 0.79 | 99.0 | 94.5 | 93.50% | 91.2 | 92. 10% | 85.54 | 86.4% |
| Chitosan hydrolysate | 0.25% | -18 | 0.74 | 99.2 | 94. 8 | 95.60% | 92.1 | 92.80% | 85.31 | 86.0% |
| Tea polyphenol | 0.01% | -20 | 0.75 | 99.0 | 95.0 | 96.00% | 92.1 | 93.00% | 85.64 | 86.5% |
| Bamboo leaf antioxidant | 0.05% | -55 | 0.75 | 99.1 | 96.2 | 97.00% | 92.4 | 93.10% | 86.30 | 87.0% |
| Rosemary extract | 0.03% | -42 | 0.75 | 99.4 | 93.9 | 94.50% | 90.5 | 91.00% | 84.49 | 85.0% |
| Liquorice antioxidant extract | 0.02% | -34 | 0.75 | 99.3 | 94. 3 | 95.00% | 91.4 | 92.00% | 85.40 | 86.0% |
| 4-hexyl-1.3-benzenediol | 0.01% | -10 | 0.75 | 99.5 | 94.1 | 94.60% | 88.6 | 89.00% | 80.60 | 81.0% |
| Dilauiylthiodipiopionate | 0.02% | -18 | 0.75 | 99.1 | 93.5 | 94.30% | 87.2 | 88.00% | 80.27 | 81.0% |
| Sodium lactate | 2% | 40 | 0.73 | 99.3 | 87.4 | 88.00% | 81.4 | 82.00% | 68.52 | 69.0% |
| Calcium lactate | 2% | 45 | 0.73 | 99.3 | 81.4 | 82.00% | 80.4 | 81.00% | 69.51 | 70.0% |
| Superoxide dismutase | 10U/ml | -30 | 0.75 | 99.0 | 94.1 | 95.00% | 88.1 | 89.00% | 80.19 | 81.0% |
| Glucose oxidase | 5U/ml | -10 | 0.75 | 99.1 | 93.6 | 94.50% | 87.2 | 88.00% | 79.28 | 80.0 % |

As shown in Table 7, when one or more redox potential regulators were used, the liquid transglutaminase had a preservation rate of enzyme activity of above 80% after storage at room temperature for 180 days, which had completely met the requirements for industrial production and application. In view of the production cost and people's desire for green natural food, potential regulators from natural sources, such as L-ascorbic acid and a salt thereof, L-serine and a salt thereof, L-cysteine and a salt thereof, reduced glutathione, tea polyphenol, soybean protein hydrolysate, wheat protein hydrolysate, casein hydrolysate, chitosan hydrolysate, bamboo leaf antioxidant, rosemary extract, and liquorice antioxidant extract, were preferred; or low-cost superoxide dismutase, glucose oxidase, sodium bisulfite, sodium metabisulfite, potassium metabisulfite, sodium sulfite, sodium hyposulfite, phytic acid, etc., were preferred. From the results as shown in Table 6 and Table 7, the redox potential regulator was determined to be added in a range of 0.0075-1%.

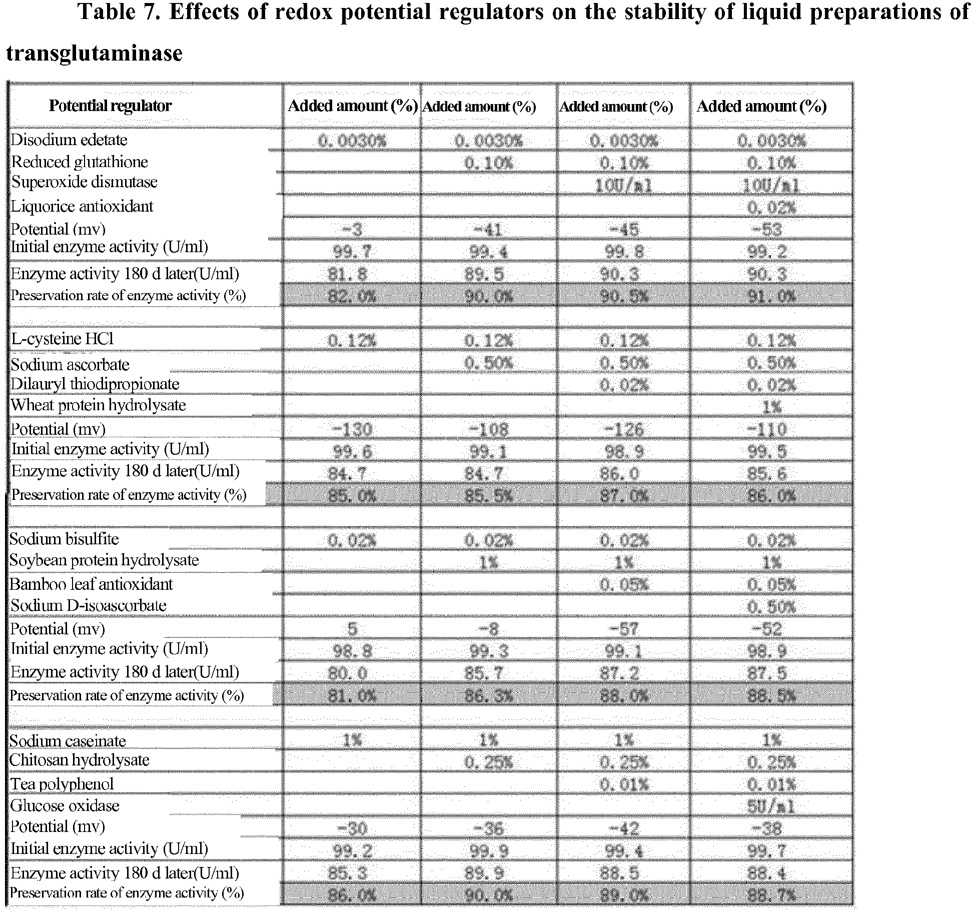

By adding a pH regulator, a water activity regulator, and a redox potential regulator, the liquid transglutaminase was adjusted to within a range of a pH of 5.0-9.0, a water activity of <0.89, and a redox potential of -400 mv to +50 mv. The liquid enzyme preparation achieved a preservation rate of above 80% after storage at room temperature under a sterile condition for 180 days, which substantively meets the requirements on a stable liquid preparation of transglutaminase that is commercially applicable.

Next, as required in GB25594-2010, an enzyme preparation for use in food industry has to meet the corresponding hygiene standards such as microorganism indexes.

Therefore, water activity, the food preservative, and the preparation methods such as the sterile filtration and filling were further investigated for their effects on the total number of colonies and harmful microorganism indexes in a liquid preparation of transglutaminase.

### 7. Effect of different concentrations of the water activity regulators on the total number of colonies of a liquid transglutaminase stored at room temperature

The pH of the concentrated transglutaminase solution was adjusted to 6.0, and 1% wheat protein hydrolysate (w/v) was added to adjust the redox potential, glycerol was added in different amounts % (w/v), and a pH regulator was added to reach a volume of 100%, obtaining a liquid enzyme preparation with different water activities. The liquid enzyme preparation was dispensed and packaged under open conditions, and changes in the total number of colonies in the enzyme preparation were investigated during storage at room temperature. The results were shown in Table 8.

**Table 8. Effects of different concentrations of water activity regulators on the total numbers of colonies in a liquid enzyme preparation**

| **Glycerol (%)** | **Water activity** | **Total number of colonies (CFU/ml)** | | | **Evaluation** |
|---|---|---|---|---|---|
| | | **30 d** | **60 d** | **180 d** | |
| 30 | 0.89 | 3800 | >10⁶ | >10⁷ | **unqualified** |
| 40 | 0.85 | 2800 | 4000 | 4230 | **qualified** |
| 50 | 0.80 | 2950 | 3200 | 3850 | **qualified** |
| 60 | 0.75 | 2880 | 3150 | 3180 | **qualified** |
| 70 | 0.62 | 2450 | 2960 | 3050 | **qualified** |
| 80 | 0.52 | 2760 | 2880 | 2940 | **qualified** |
| 90 | 0.31 | 2670 | 2830 | 2990 | **qualified** |

The data in Table 8 showed that, when the water activity was greater than 0.85, glycerol was present in an amount of below 40%, and no preservative was present in the liquid enzyme preparation of transglutaminase, microorganisms proliferated rapidly, and the total number of colonies went beyond the maximum number as specified in relevant laws and regulations during the storage for 180 days at room temperature. When the water activity regulator was present in an amount of greater than 40%, the growth and proliferation of microorganisms were substantially inhibited due to the low water activity itself. In view of the requirements on hygienic indexes of enzyme preparations for use in food industry, formulas with a water activity regulator of above 40%-80% were preferred.

### 8. Effects of different food preservatives on microorganisms in an enzyme solution

A transglutaminase solution was diluted with 0.02 M sodium citrate buffer (pH 6.0) to 1000 u/ml. To 100 ml diluted enzyme solution, 300g glycerol and 10g a potential regulator of wheat protein hydrolysate and then 0.02 M sodium citrate buffer, pH 6.0 was added to a final volume of 1000 ml. The food preservatives as listed in GB2760-2014 were added as shown in Table 9, the controls were A: no preservative, and B: no preservative, but the solution was filtered through a 0.1-0.22µm membrane and filled under sterile conditions. Under open conditions, the enzyme solution as experimental sample was filled in PET opaque bottles, and placed at room temperature for 180 days. In accordance with GB4789.2-2010 National Food Safety Standard "Food microbiological examination: Aerobic plate count", GB 4789.3 National Food Safety Standard "Food microbiological examination: Enumeration of coliforms", GB/T 4789.38 National Food Safety Standard "Food microbiological examination: *Escherichia coli* count", and GB 4789.4-2010 National Food Safety Standard "Food microbiological examination: *Salmonella",* microbiological examination was carried out. The results were shown in Table 9.

**Table 9. Effects of different food preservatives on microorganisms in liquid enzyme preparations**

| Treatment | Total number of colonies (CFU/mL) | Coliforms (CFU/mL) | *Escherichia coli* (CFU/mL) | *Salmonella* (25g) | Detection result |
|---|---|---|---|---|---|
| No addition of preservative A | >10⁹ | >1000 | not detected | not detected | Smelly, unqualified |
| Bacteria removal by 0.1 µm membrane B | not detected | not detected | not detected | not detected | qualified |
| 0.05% Benzoic acid | 3220 | 10 | not detected | not detected | qualified |
| 0.1% Sodium benzoate | 4800 | 10 | not detected | not detected | qualified |
| 0.25% Propionic acid | 4000 | 5 | not detected | not detected | qualified |
| 0.25% Sodium propionate | 4200 | 5 | not detected | not detected | qualified |
| 0.025% Dimethyl dicarbonate | 2400 | 7 | not detected | not detected | qualified |
| 0.05% Potassium sorbate | 2500 | 11 | not detected | not detected | qualified |
| 0.05% Sodium bisulfite | 2690 | 9 | not detected | not detected | qualified |
| 0.02% Ethyl lauroyl arginate HCl | 2150 | 8 | not detected | not detected | qualified |
| 0.03% Sodium dehydroacetate | 2810 | 11 | not detected | not detected | qualified |
| 0.05% Sodium diacetate | 3040 | 9 | not detected | not detected | qualified |
| 0.02% Nipagin complex esters | 1200 | 6 | not detected | not detected | qualified |
| 0.02% Sodium metabisulphite | 2860 | 8 | not detected | not detected | qualified |
| 0.05% Potassium metabisulphite | 2970 | 10 | not detected | not detected | qualified |
| 0.02% ε-polylysine | 3150 | 12 | not detected | not detected | qualified |
| 0.02% ε-polylysine HCl | 3400 | 11 | not detected | not detected | qualified |
| 0.03% Natamycin | 3450 | 8 | not detected | not detected | qualified |
| 0.05% Lysozyme | 1200 | not detected | not detected | not detected | qualified |
| 0.02% Nisin | 1800 | 18 | not detected | not detected | qualified |

The data in Table 9 showed that, when the water activity regulator was at a concentration as low as 30% (w/v), the various food preservatives added at the amounts permissive under the laws and regulations can effectively inhibit the proliferation of microorganisms, thereby ensure the hygiene and safety of a transglutaminase solution. In view of customer preference and the applied range of various food preservatives, one food preservative or a combination of several food preservatives could be used in the production of a liquid transglutaminase; preferably natural food preservatives, such as ε-polylysine, natamycin, lysozyme, and Nisin. Or the liquid can be filtered through a 0.1 µm membrane followed by sterile filling. Or alternatively, in view of the application cost, one or more of sorbic acid and potassium sorbate, sodium diacetate, sodium dehydroacetate, methyl p-hydroxybenzoate, potassium metabisulfite, and sodium metabisulfite may be used; or filtration through a 0.1 µm membrane followed by sterile filling may be employed in the process.

### 9. Effects of different enzyme activities on stability of liquid preparations at room temperature

To different amounts of purified concentrated enzyme solutions, 50% (w/v) glycerol, and 0.1% (w/v) sodium bisulfite were added, and a pH regulator was added to a final volume of 1000 ml. After sterile filtration, the resultant solution was placed at room temperature for 180 days, and then the enzyme activity was determined and the preservation rate of enzyme activity (%) was compared.

**Table 10. Effects of different enzyme activities of enzyme solutions on the stability of a liquid transglutaminase**

| **Added amount of enzyme solution% (v/v)** | **Added volume of enzyme solution (ml)** | **Enzyme activity after adjustment (u/ml)** | **Enzyme activity 180 d later (u/ml)** | **Preservation rate of enzyme act**i**vity %** |
|---|---|---|---|---|
| 0.4% | 4ml | 10.2 | 10.1 | 99.02 |
| 2% | 20ml | 50.1 | 49. 2 | 98.20 |
| 4% | 40ml | 99.9 | 98.2 | 98.30 |
| 8% | 80ml | 200.1 | 197.2 | 98.55 |
| 20% | 200ml | 498.8 | 495.8 | 99.40 |
| 30% | 300ml | 749.9 | 742.3 | 98.99 |
| 40% | 400ml | 1000.0 | 991.3 | 99.13 |
| 45% | 450ml | 1124.5 | 1112.4 | 98.92 |

As shown in Table 10, the effects of different enzyme activities of enzyme solutions on the stability of a liquid transglutaminase were not significant. However, in the practical applications, it is preferable to control the enzyme activity of a liquid preparation within the range of 10-1000 u/ml.

By investigating the effects of the above-mentioned physicochemical factors on the preservation rate of enzyme activity (stability) of a transglutaminase solution at room temperature, the inventors determined the optimal pH range, the kinds of pH regulators, the range of water activity and the amount of a water activity regulator, the redox potential range and the kinds of redox potential regulators, which can be used/applied to keep the liquid preparation of transglutaminase stable at room temperature.

Furthermore, it was verified that by means of sterile filtration and filling or addition of a preservative in the production, a liquid enzyme preparation can be stably stored at room temperature.

Based on the principle that several methods are used in combination to enhance enzyme stability so as to achieve the purpose of synergistically enhancing enzyme stability, the invention successfully solves the problem concerning the storage of a liquid preparation of transglutaminase at room temperature by controlling the intermediate indexes and parameters during production, and develops a liquid preparation of transglutaminase that has good stability at room temperature, can be stored for a long time, and can be produced in a commercial scale. It is of great significance in reducing production cost for transglutaminase, energy conservation, environment protection, healthy use, and market promotion.

To sum up, the technical solutions of the invention are as follows:
a liquid enzyme preparation, characterized in that the liquid enzyme preparation is a liquid enzyme preparation of transglutaminase EC2.3.2.13 having an enzyme activity of 10-1000 u/ml;
a liquid enzyme preparation, characterized in that its components and amounts thereof are as follows: the liquid preparation of transglutaminase has an enzyme activity of 10-1000 u/ml, a water activity regulator is present in an amount of 30-80 w/v%, a redox potential regulator is present in an amount of 0.0075-1 w/v%, a food preservative is present in an amount of 0-0.1 w/v%, and a pH regulator is added to a final volume of 100%.

The liquid enzyme preparation has the following physicochemical properties:
1) having a pH of 5.0-9.0; preferably a pH of 5.0-7.5
2) having a water activity A_{w} of ≤0.89; preferably a water activity A_{w} of 0.60-0.85; and
3) having a redox potential of -400mv to +50mv; preferably, a redox potential of -400 mv to 0 mv. the pH regulator is one of hydrochloric acid, sulfuric acid, acetic acid, lactic acid, citric acid, malic acid, phytic acid, phosphoric acid, nitric acid, oxalic acid, sodium hydroxide, potassium hydroxide, sodium carbonate, sodium bicarbonate, potassium carbonate, potassium bicarbonate, trisodium citrate, tripotassium citrate, sodium acetate, potassium acetate, sodium lactate, potassium lactate, disodium hydrogen phosphate, dipotassium hydrogen phosphate, sodium phosphate, potassium phosphate, tap water, purified water, and mineral water, or a buffer system consisting of several pH regulators; preferably an regulator having a pH buffer capacity, such as a buffer comprising one of phosphoric acid, acetic acid, lactic acid or citric acid, or a salt thereof.
   the water activity regulator is selected from the group comprising of sorbitol, maltitol, propylene glycol, glycerol, xylitol, PEG, trehalose, sucrose, maltose, isomaltose, maltodextrin, xylitol, and mannitol, preferably comprising sorbitol, maltitol, or glycerol, or a combination thereof. The amount is 30-80% (w/v), preferably 30-70% (w/v).

The redox potential regulator comprises one or more of L-ascorbic acid and a salt thereof, L-serine and a salt thereof, L-cysteine and a salt thereof, reduced glutathione, tea polyphenol, soybean protein hydrolysate, wheat protein hydrolysate, casein hydrolysate, chitosan hydrolysate, bamboo leaf antioxidant, rosemary extract, liquorice antioxidant extract, superoxide dismutase, glucose oxidase, sodium bisulfite, sodium metabisulfite, potassium metabisulfite, sodium sulfite, sodium hyposulfite, phytic acid, etc., and is used in an amount of 0.0075%-1%.

The food preservative is selected from the group comprising of ε-polylysine, natamycin, lysozyme, Nisin, potassium sorbate, sodium dehydroacetate, sodium diacetate, methyl p-hydroxybenzoate, ethyl lauroyl arginate HCl, potassium metabisulfite, and sodium metabisulfite, and is generally added in an amount of 0%-0.1%.

A method for preparing a liquid enzyme preparation, characterized by the following steps:
1) purification of an enzyme solution: subjecting a crude enzyme solution to pressure filtration, microfiltration, and secondary ultrafiltration, to obtain a purified concentrated enzyme solution;
2) mixing: weighing the purified concentrated enzyme solution, a water activity regulator, and a redox potential regulator quantitatively, adding a food preservative if present, and mixing; adding a pH regulator to a final volume of 100%; after mixing homogeneously, testing and adjusting the liquid enzyme preparation for the following parameters: a pH of 5.0-9.0, a water activity A_{w} of ≤0.89, and a redox potential of -400mv to +50mv;
3) bacteria removal: filtering the resulting mixture through a 0.1-0.22µm membrane for sterilization, followed by sterile filling;
4) package: packaging the liquid enzyme preparation by sterile filling or another relevant liquid package process.

The invention for the first time achieved a liquid preparation of transglutaminase that has an enzyme activity not less than 80% and is stable in the form of solution after storage at room temperature for 6 months, meets the requirements in market commercialization, is convenient for production, has good application effect, and does not need refrigeration or cold-chain transportation.

### Inventive step of the invention:

The invention for the first time achieved a liquid preparation of transglutaminase that has a preservation rate of enzyme activity of above 80% and is stable in the form of solution after storage at room temperature for 6 months, meets the requirements in market commercialization, is convenient for production, has good application effect, and does not need refrigeration or cold-chain transportation. The invention has an important significance in promoting the market application and development of transglutaminase.

The invention for the first time found that the redox potential in a system is the most important factor for maintaining a liquid transglutaminase stable at room temperature. When the redox potential of the liquid enzyme preparation is between (-400mv) and +50mv, the stability of the transglutaminase solution at room temperature is greatly enhanced. Transglutaminase, as a macromolecular protein having catalytic activity, has to maintain the stability of its three-dimensional structure to the largest extent in order to maintain the stability of the enzyme molecules. However, the groups and fragments inside the macromolecule have different charge characteristics due to the difference in their amino acid sequences. In a liquid system, it exhibits a typical amphoteric electrolyte character. Since a three-dimensional structure needs to be maintained by noncovalent bonds such as van der Waals force among groups and fragments, and such weak charge action is easily influenced by the redox potential of the liquid system in which the enzyme molecules are present. When the redox potential of a liquid system is higher than the steady potential of the enzyme molecules, transglutaminase molecules as reductant are oxidized in the system; when the redox potential of the liquid system is lower than the steady potential of the enzyme molecules, the transglutaminase molecules as oxidant are reduced in the system, resulting in the loss of the three-dimensional structure and activity of the enzyme molecules. In the invention, a liquid preparation of transglutaminase being stable at room temperature was obtained, by adjusting the potential of the liquid enzyme preparation to be within the above-mentioned potential range with a suitable redox potential regulator, wherein the redox potential of the environment where transglutaminase is present is just in the range in which the enzyme molecule is stable.

Therefore, in the formula of the invention, a redox potential regulator was added for the first time, such as reduced glutathione, L-cysteine and hydrochloride thereof, wheat protein hydrolysate, chitosan hydrolysate, bamboo leaf antioxidant, rosemary extract, liquorice antioxidant extract, and sulfite, which substantively ensures the stability of a liquid preparation of transglutaminase at room temperature, and is key in the formula.

In the formula of the invention, a water activity regulator and a pH regulator are also used, wherein a buffer can effectively buffer the change in the pH of a liquid enzyme preparation caused by the addition of various additives, and keep the pH of the liquid transglutaminase in the optimal range. The addition of a water activity regulator can keep the liquid enzyme preparation in a lower water activity A_{w}, and is also favorable for inhibiting the proliferation of microorganisms and enhancing the steric stability of the enzyme molecules. For example, water activity regulators comprising abundant hydroxyl units, such as trehalose, PEG, and sorbitol, are used in the invention, which can greatly stabilize the steric structure of the transglutaminase molecules. The use of a water activity regulator, a redox potential regulator, and a pH regulator in combination can synergistically maintain the enzyme stability, and achieve the best stabilizing effect.

In the preparation process of the invention, a liquid preparation of transglutaminase is prepared by means of mixing. Firstly, a concentrated transglutaminase solution or transglutaminase powder is purified. Since transglutaminase itself has a molecular weight of about 38-45 KDa, pressure filtration, and microfiltration (with a pore size of 0.1-1µm) are carried out first. This step can remove precipitates such as bacterial solids and particles. Then, the resultant solution is subjected to ultrafiltration (molecular weight cutoff: 100-200 KDa), and this step can remove most of microorganisms, mitigate microbial spoilage, and reduce the effect of microorganisms on the enzyme. Finally, ultrafiltration (molecular weight cutoff: 10-30 KDa) is carried out to obtain a purified concentrated enzyme solution. Next, a water activity regulator, a redox potential regulator, and a food preservative are added in accordance with the formula, a pH regulator is used to adjust the pH. After homogeneously mixing, the relevant parameters are determined. Slight adjustment is performed by adding regulators, so as to meet the physicochemical properties of the preparation. The mixed solution is further subjected to membrane filtration to remove bacteria, followed by filling, so that the whole solution is in a sterile environment or an environment with little bacteria. Meanwhile, a food preservative may be optionally used to prevent growth of bacteria in the liquid enzyme preparation during storage and transportation. The formula and the preparation method according to the invention can greatly enhance the thermal stability of transglutaminase, and prolong the shelf life of the liquid enzyme preparation.

The components used in the formula of the liquid preparation of transglutaminase according to the invention are cheap and can be obtained easily, and their food grade sources are available. The formula of the liquid preparation of transglutaminase also meets the national food safety and food additive standards. The process for preparing a liquid enzyme preparation is scientific and reasonable, and has a high efficiency in production. It is greatly simplified in terms of process and production cost as compared to the process of preparing a powder enzyme preparation by lyophilization. There is almost no loss in enzyme activity, and the production efficiency is greatly enhanced. It is in line with the concepts of energy conservation, environmental protection, healthy use of enzymes, and green economy as promoted in China.

### Description of the drawing

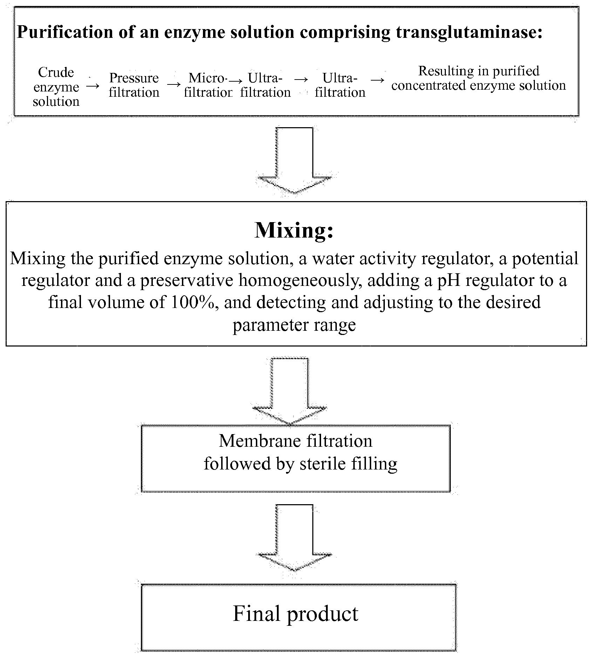

Figure 1 is a scheme of the preparation procedure for a liquid enzyme preparation.

### Detailed embodiments of the invention

The following examples are provided in order to better understand the invention, the scope as claimed in the invention includes, but is not limited to, the contents described in the following examples, and any modifications and improvements made according to conventional knowledge in the art will be within the scope as claimed in the invention. The scheme is shown in Figure 1.

### Example 1

A crude transglutaminase solution was prepared by means of microbiological fermentation, which could be carried out by reference to the Patent (Patent Publication No: EP0379606B1): *Streptomyces mobaraensis* was used as the original strain, a few bacterial colonies in a state of good growth were picked with an inoculating loop, inoculated on a slant culture medium, and cultured at a constant temperature of 30 °C for 7 days. The activated strains were further inoculated in a seed culture medium, cultured at 30 °C for 48 h, added to a fermentation medium in an inoculation amount of 10%, and cultured at 30 °C for 48-72 h, thereby obtaining a crude transglutaminase solution. The crude enzyme solution was purified and concentrated by the following steps:
1) pressure filtration: at a pore size of 1 µm, an operating pressure of 0.20MPa, and a temperature of 20 °C, obtaining a filtrate;
2) microfiltration: at a pore size of 0.25 µm, an operating pressure of 0.25MPa, and a temperature of 20 °C, obtaining a filtrate;
3) ultrafiltration: at a molecular weight cutoff of 100KDa, an operating pressure of 0.30MPa, and a temperature of 20 °C, obtaining a filtrate;
4) ultrafiltration: at a molecular weight cutoff of 10KDa, an operating pressure of 0.30MPa, and a temperature of 20 °C, keeping the retenate (i.e. a purified concentrated enzyme solution).

The enzyme activity was determined by hydroxamic acid method (Peng Can, Stabilization Study of Microbial Transglutaminase [D]. East China Normal University, 2007), (the same below), and a purified concentrated transglutaminase solution having an enzyme activity of 2500 u/ml was obtained. By measurement, it had a pH of 5.80, water activity of 0.95, and a redox potential of 60mv. The solution is kept for later use.

### Example 2

A crude transglutaminase solution was prepared by dissolving enzyme powder. A certain amount of pure water was weighed, and placed in a container equipped with a stirrer. A transglutaminase powder with an enzyme activity of 5000-8000 u/ml (an enzyme powder produced by Shanghai Kinry Food Ingredients Co., Ltd) was added slowly with stirring, so as to obtain a dissolved enzyme solution at a certain concentration. Bacteria were removed by microfiltration to obtain a clear transglutaminase solution with an enzyme activity of 2500 u/ml, i.e. a purified concentrated transglutaminase solution with an enzyme activity of 2500 u/ml. By measurement, it had a pH of 6.30, water activity of 0.95, and a redox potential of 58mv. The solution is kept for later use.

### Example 3

A method for preparing a liquid transglutaminase: the liquid enzyme in a total volume of 1000 ml was prepared as followed, and the raw materials were weighed in accordance with Table 11.

**Table 11. Preparation of a liquid transglutaminase**

| Formula | | | |
|---|---|---|---|
| Composition | Component | Addition level % | Added amount |
| Transglutaminase | Purified enzyme solution (Example 1) | 20%(v/v) | 200ml |
| Water activity regulator | Glycerol | 50%(w/v) | 500g |
| Potential regulator | L-cysteine | 0.15%(w/v) | 1.5g |
| pH regulator | 0.02M pH6.0 Sodium citrate buffer | / | Adjusted to the final volume of 1000ml |

The raw materials of the formula were weighed and mixed homogeneously, and after filtration through a 0.1 µm sterile membrane, a light-yellow solution was obtained. By measurement, it had a redox potential of -100 mv, water activity of 0.79, a pH of 6.0, and an enzyme activity of 498.1 u/ml. It was filled in ten of 100mL PET opaque bottles, to finally obtain a transglutaminase preparation in a liquid form. After storage at room temperature for 180 days, the enzyme preparation of the formula was observed, the enzyme activity was measured, and the preservation rate of enzyme activity was calculated. By reference to GB4789.2-2010 National Food Safety Standard "Food microbiological examination: Aerobic plate count", GB 4789.3 National Food Safety Standard "Food microbiological examination: Enumeration of coliforms", GB/T 4789.38 National Food Safety Standard "Food microbiological examination: *Escherichia coli* count", and GB 4789.4-2010 National Food Safety Standard "Food microbiological examination: *Salmonella",* microbiological examination was carried out. The results showed that the liquid enzyme preparation had no significant change in appearance, flavor and the like. It had a preservation rate of enzyme activity of 86%, and its microorganism indexes meet the requirements in GB25594-2010 Standard. Therefore, it could be used for commercialization.

### Example 4

The purified concentrated transglutaminase solution prepared in Example 2 was used to prepare a liquid preparation of transglutaminase in accordance with the formula in Table 12. After mixing homogenously, the pH, water activity, redox potential, and initial enzyme activity were measured in accordance with the relevant methods as described in Example 3. The liquid preparation of transglutaminase was then filtrated through 0.1 µm sterile membrane, and was dispensed and packaged in PET bottles under sterile condition. After storage at room temperature for 180 days, the appearance of the liquid preparation of transglutaminase was observed, and the endpoint enzyme activity, microorganism indexes and the like were measured in accordance with the relevant methods as described in Example 3, and the preservation rate of enzyme activity was calculated.

**Table 12. Preparation of a liquid transglutaminase**

| Formula | | | |
|---|---|---|---|
| Composition | Component | Addition level % | Added amount |
| Transglutaminase | Purified enzyme solution (Example 2) | 4%(v/v) | 4L |
| Water activity regulator | Glycerol | 30%(w/v) | 30kg |
| Potential regulator | Reduced glutathione | 0.1%(w/v) | 100g |
| pH regulator | 0.02M pH6.0 Sodium citrate buffer | / | Adjusted to the final volume of 100L |

The results showed that the transglutaminase preparation prepared by the method had good stability, had no significant and visible change in appearance, and had a preservation rate of enzyme activity of 82%. The microorganism indexes met the requirements in GB25594-2010 Standard. It could be used for commercialization. The use of filtration through 0.1 µm sterile membrane and sterile filling could ensure the liquid enzyme preparation to have a preservation rate of enzyme activity of up to 80%, and could control the microorganism indexes in the liquid enzyme preparation of a formula with 30% water activity regulator during the period of storage.

### Example 5

**Table 13. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 99.5u/ml | Appearance: No visible change |
| Transglutaminase | Purified concentrated enzyme solution (Example 2) | 4%(v/v) | 4L | pH: 6.0 | |
| Water activity regulator | Glycerol | 30%(w/v) | 30kg | Water activity: 0.89 | Preservation rate of enzyme activity: 84% |
| Potential regulator | Reduced glutathione | 0.1%(w/v) | 100g | | |
| Preservative | Sodium dehydroacetate | 0.03%(w/v) | 30g | Redox potential: -35mv | Microorganism index: qualified |
| pH regulator | 0.02M pH6.0 Sodium citrate buffer | / | Adjusted to the final volume of 100L | | |

After mixing said raw materials homogeneously, the mixture was directly filled into PET bottles under open environments. After storage at room temperature for 180 days, the transglutaminase preparation prepared by the method had good stability, had no significant and visible change in appearance, and had a preservation rate of enzyme activity of 84%. The microorganism indexes met the requirements in GB25594-2010 Standard, and it could be used for commercialization.

### Example 6

**Table 14. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 101u/ml | Appearance: No visible change |
| Transglutaminase | Purified concentrated enzyme solution (Example 1) | 4%(v/v) | 4L | pH: 6.0 | |
| Water activity regulator | Glycerol 70%-maltitol 30% | 40%(w/v) | 40kg | Water activity: 0.85 | Preservation rate of enzyme activity: 87% |
| Potential regulator | Reduced glutathione | 0.1 %(w/v) | 100g | | |
| Preservative | Sodium dehydroacetate | 0.03%(w/v) | 30g | Redox potential: - 37mv | Microorganism index: qualified |
| pH regulator | 0.02M pH6.0 Sodium citrate buffer | / | Adjusted to the final volume of 100L | | |

The components as listed in Table 14 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 87%, the microorganism indexes met the requirements, and thus a stable liquid preparation of transglutaminase was obtained.

### Example 7

**Table 15. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 111u/ml | Appearance: No visible change |
| Transglutaminase | Purified concentrated enzyme solution (Example 1) | 4.4%(v/v) | 4.4L | pH: 6.7 | |
| Water activity regulator | Glycerol 70%- Sorbitol 30% | 50%(w/v) | 50kg | Water activity: 0.85 | Preservation rate of enzyme activity: 89% |
| Potential regulator | Wheat protein hydrolysate | 1%(w/v) | 1kg | | |
| Preservative | ε-polylysine | 0.02%(w/v) | 20g | Redox potential: -32mv | Microorganism index: qualified |
| pH regulator | Tap water | / | Adjusted to the final volume of 100L | | |

The components as listed in Table 15 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, in the presence of a preservative, after storage at room temperature for 180 days, the preservation rate of enzyme activity reached 89%, and the microorganism indexes met the requirements, thus a stable liquid preparation of transglutaminase was obtained.

### Example 8

The components as listed in Table 16 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 88%, and the microorganism indexes met the requirements, thus a stable liquid preparation of transglutaminase was obtained, where the potential regulators and the water activity regulators were combined components, and a preservative was added.

**Table 16. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 101u/ml | Appearance: No visible change |
| Transglutaminase | Purified concentrated enzyme solution (Example 1) | 4%(v/v) | 4L | pH: 6.6 | |
| Water activity regulator | Glycerol 70% Sorbitol 30% | 50%(w/v) | 50kg | Water activity: 0.83 | Preservation rate of enzyme activity: 89% |
| Potential regulator | Sodium caseinate | 1.0%(w/v) | 1kg | | |
| | Chitosan hydrolysate | 0.25%(w/v) | 250 g | | |
| | Tea polyphenol | 0.01%(w/v) | 10g | | |
| | Glucose oxidase | / | 5u/ml | | |
| Preservative | Lysozyme | 0.5%(w/v) | 50g | Redox potential: -38mv | Microorganism index: qualified |
| pH regulator | Tap water | / | Adjusted to the final volume of 100L | | |

### Example 9

**Table 17. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 1000 u/ml | Appearance: No visible change |
| Transglutaminase | Purified concentrated enzyme solution (Example 1) | 40%(v/v) | 40L | pH: 6.2 | |
| Water activity regulator | Glycerol | 50%(w/v) | 50kg | Water activity: 0.84 | Preservation rate of enzyme activity: 88% |
| Potential regulator | Sodium caseinate | 1.0%(w/v) | 1kg | | |
| | Chitosan hydrolysate | 0.25%(w/v) | 250 g | | |
| | Tea polyphenol | 0.01%(w/v) | 10g | | |
| | Glucose oxidase | / | 5u/ml | | |
| Preservative | Lysozyme | 0.5%(w/v) | 50g | Redox potential: -42mv | Microorganism index: qualified |
| pH regulator | Purified water | / | Adjusted to the final volume of 100L | | |

The components as listed in Table 17 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 88%, and the microorganism indexes met the requirements, thus a stable liquid preparation of transglutaminase with an enzyme activity of 1000 u/ml was obtained.

### Example 10

The components as listed in Table 18 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 81.5%, and the microorganism indexes met the requirements, thus a stable liquid preparation of transglutaminase with an enzyme activity of 10 u/ml and a pH of 5.5 was obtained.

**Table 18. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 10 u/ml | Appearance: No visible change |
| Transglutaminase | Purified concentrated enzyme solution (Example 1) | 0.4%(v/v) | 0.4 L | pH: 5.5 | |
| Water activity regulator | Glycerol | 50%(w/v) | 50kg | Water activity: 0.83 | Preservation rate of enzyme activity: 81.5% |
| Potential regulator | Sodium bisulfite | 0.02%(w/v) | 20g | | |
| | Soybean protein hydrolysate | 1%(w/v) | 1kg | | |
| | Bamboo leaf antioxidant | 0.05%(w/v) | 50g | | |
| | Sodium D-isoascorbate | 0.5% (w/v) | 500g | | |
| Preservative | Methyl parahydroxybenzoate | 0.25%(w/v) | 250g | Redox potential: -60mv | Microorganism index: qualified |
| pH regulator | 0.1M HCl Purified water | 0.1% (v/v) / | 0.1L Adjusted to the final volume of 100L | | |

### Example 11

The components as listed in Table 19 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 81%, and the microorganism indexes met the requirements, thus a stable liquid preparation of transglutaminase with an enzyme activity of 99.8 u/ml and a pH of 8.0 was obtained.

**Table 19. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 99.8 u/ml | Appearance: No visible change |
| Transglutaminase | Purified concentrated enzyme solution (Example 1) | 4%(v/v) | 4 L | pH: 8.0 | |
| Water activity regulator | Glycerol | 50%(w/v) | 50kg | Water activity: 0.83 | Preservation rate of enzyme activity: 80.5% |
| Potential regulator | Wheat protein hydrolysate | 1.0%(w/v) | 1kg | | |
| Preservative | Sodium diacetate | 0.5%(w/v) | 500g | Redox potential: -32mv | Microorganism index: qualified |
| pH regulator | NaOH Purified water | 0.05% (w/v) / | 50g Adjusted to the final volume of 100L | | |

### Example 12

The components as listed in Table 20 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 81%, and the microorganism indexes met the requirements, thus a stable liquid preparation of transglutaminase with an enzyme activity of 100 u/ml, a pH of 6.0 and a redox potential of -400 mv was obtained. Since sodium borohydride in the preparation does not meet the requirement in Food Hygiene Regulations, the liquid enzyme preparation of this formula can only be applied for the purpose of scientific research or non-food addition.

**Table 20. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 100 u/ml | Appearance: No visible change |
| Transglutaminase | Purified concentrated enzyme solution (Example 1) | 4%(v/v) | 4 L | pH: 6.0 | |
| Water activity regulator | Glycerol | 50%(w/v) | 50kg | Water activity: 0.84 | Preservation rate of enzyme activity: 80.1% |
| Potential regulator | Sodium borohydride | 0.055%(w/v) | 55g | | |
| Preservative | Sodium diacetate | 0.05%(w/v) | 50g | Redox potential: -400mv | Microorganism index: qualified |
| pH regulator | NaOH Purified water | 0.005% (w/v) / | 5g Adjusted to the final volume of 100L | | |

### Example 13

The components as listed in Table 21 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 82%, and the microorganism indexes met the requirements, thus a stable liquid preparation of transglutaminase with an enzyme activity of 50 u/ml, a pH of 6.0 and a redox potential of 50 mv was obtained.

**Table 21. Preparation of a liquid transglutaminase**

| Formula | | | | Physicochemical parameters of the enzyme preparation | Results 180 d later |
|---|---|---|---|---|---|
| Composition | Component | Addition level % | Added amount | Enzyme activity: 50 u/ml | Appearance: No visible change |
| Transglutaminase | Purified concentrated enzyme solution (Example 1) | 2%(v/v) | 2 L | pH: 6.0 | |
| Water activity regulator | Glycerol | 50%(w/v) | 50kg | Water activity: 0.85 | Preservation rate of enzyme activity: 82% |
| Potential regulator | Wheat protein hydrolysate | 0.05%(w/v) | 50g | | |
| | L-cysteine HCl | 0.01%(w/v) | 10g | | |
| Preservative | Lysozyme | 0.05%(w/v) | 50g | Redox potential: 50mv | Microorganism index: qualified |
| pH regulator | 0.1M pH 6.0 Phosphate buffer | / | Adjusted to the final volume of 100L | | |

### Example 14

**Table 22. Preparation of a liquid transglutaminase**

| Formula | | | | Physicochemical parameters | Results 180 d later |
|---|---|---|---|---|---|
| Composition | Component | Addition level % | Added amount | Enzyme activity: 100u/ml | Appearance: No visible change |
| Transglutaminase | Purified concentrated enzyme solution (Example 1) | 4%(v/v) | 4L | pH. 6.0 | |
| Water activity regulator | Glycerol | 50%(w/v) | 50kg | Water activity: 0.85 | Preservation rate of enzymatic activity: 84% |
| Potential regulator | Vheat protein hydrolysate | 0.1%(w/v) | 100g | | |
| | L-cysteine HCl | 003%(w/v) | 30g | | |
| Preservative | Lysozyme | 0 05%(w/v) | 50g | Redox potential: 0mv | Microorganism index: qualified |
| pH regulator | 0.02MpH6.0 Phosphate buffer | / | Adjusted to the final volume of 100L | | |

The components as listed in Table 22 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 84%, and the microorganism indexes met the requirements, thus a stable liquid preparation of transglutaminase with an enzyme activity of 100 u/ml, a pH of 6.0 and a redox potential of 0 mv was obtained.

### Example 15

The components as listed in Table 23 were mixed homogeneously to prepare a liquid preparation of transglutaminase, and the relevant parameters were measured. The liquid preparation of transglutaminase was filled into 1L PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 89%, and the microorganism indexes met the requirements, thus a stable liquid preparation of transglutaminase with an enzyme activity of 50 u/ml, water activity of 0.61, and a redox potential of -140 mv was obtained. No preservative was comprised in the formula.

**Table 23. Preparation of a liquid transglutaminase**

| Formula | | | | Physicochemical parameters | Results 180 d later |
|---|---|---|---|---|---|
| Composition | Component | Addition level % | Added amount | Enzyme activity: 50u/ml | Appearance: No visible change |
| Transglutaminase | Purified concentrated enzyme solution (Example 1) | 2%(v/v) | 2L | pH: 6.0 | |
| Water activity regulator | Glycerol | 70%(w/v) | 70kg | Water activity: 0.61 | Preservation rate of enzyme activity:89% |
| Potential regulator | L-cysteine HCl | 0.12%(w/v) | 120g | | |
| Preservative | No | - | - | Redox potential: -140mv | Microorganism index: qualified |
| pH regulator | pH6.0 Acetate buffer | / | Adjusted to the final volume of 100L | | |

### Example 16

The components in the formula of Example 15 were mixed homogeneously to prepare a liquid preparation of transglutaminase, and the relevant parameters were measured. The liquid preparation of transglutaminase was filled into 1L opaque PET bottles under open conditions. After storage at room temperature for 180 days, the appearance of the liquid enzyme preparation was observed, and the enzyme activity and microorganism indexes were measured. The results showed that, after storage at room temperature for 180 days, the preservation rate of enzyme activity reached 88%, and the microorganism indexes met the requirements, thus a stable liquid preparation of transglutaminase with an enzyme activity of 101 u/ml, water activity of 0.85, and a redox potential of -38 mv was obtained. The package for the enzyme preparation was an opaque material, and had no significant effect on the enzyme stability.

### Example 17

After storage at room temperature for 180 days, the liquid preparation of transglutaminase prepared in Example 7 was measured to have an enzyme activity of 98.79 u/ml. It was used in the production of sausage. The particular formula and process were shown in Table 24-1. A commercially available transglutaminase preparation in a powder form (from Kinry Biotech (Jinan) Co., Ltd.) was used as control. It was measured to have an enzyme activity of 100 u/g before use.

**Table 24-1. Use of liquid, powder transglutaminase in production of sausage**

| Item | Liquid enzyme preparation (A) | Powder enzyme control(B) | Control without addition of enzvme(C) |
|---|---|---|---|
| Enzyme dose | 150ml | 150g | - |
| Lean pork | 40kg | 40kg | 40kg |
| Chicken breast | 10kg | 10kg | 10kg |
| Pork fat lining | 17kg | 17kg | 17kg |
| Edible salt | 1.6kg | 1.6kg | 1.6kg |
| Composite phosphate | 250g | 250g | 250g |
| Sodium caseinate | 200g | 200g | 200g |
| Gourmet powder | 500g | 500g | 500g |
| Spice | 5kg | 5kg | 5kg |
| I+G | 20g | 20g | 20g |
| Carrageenan | 300g | 300g | 300g |
| Soy protein isolates | 1.8kg | 1.8kg | 1.8kg |
| Corn starch | 5kg | 5kg | 5kg |
| Ice water | 14kg | 14kg | 14kg |
| Sodium lactate | 1kg | 1kg | 1kg |
| Sodium isoascorbate | 30g | 30g | 30g |
| White sugar | 3kg | 3kg | 3kg |
| Pork soluble meal | 300g | 300g | 300g |
| Processing procedure | In accordance with the formula above, the raw materials, meat and fat lining, were defrosted, and then minced to a size of 0.5 cm. All the raw materials were subjected to the rolling and rubbing in a vacuum tumbler. The rolling and rubbing were performed in the following manner: rolling and rubbing for 10 min, resting for 2 min, in a total period of 60 min. The resultant meat thus obtained was poured into a sausage stuffer. After sausage stuffing was finished, the sausage was dried at 60 °C for 25 min, and cooked at 80 °C for 30 min. The quality of the product was evaluated after cooling. | | |

After the sausage was made, the final product was tested for the indexes such as gel strength, elasticity and sensory score. The experimental results were shown in Table 24-2.

**Table 24-2. Effect of a transglutaminase preparation in a liquid form and a powder form on indexes of sausage**

| Item | Sample A | Sample B | Sample C |
|---|---|---|---|
| Gel strength (g/cm²) | 463 | 396 | 244 |
| Elasticity (mm) | 2.502 | 2.071 | 1.105 |
| Cooking loss (%) | 2.8% | 2.9% | 4% |
| Sensory score (maximum score: 100) | 92.1 | 87.7 | 75.6 |

The results of experimental test showed that the liquid preparation of transglutaminase had a good effect in the sausage application, and had a significantly improved performance indexes of sausage products such as gel strength and elasticity, as compared to the powder transglutaminase preparation at the same dose. Cooling loss was somewhat but not significantly improved, and the sensory evaluation was significantly improved.

### Example 18

The liquid transglutaminase product prepared in Example 5 (99.5 u/mL) was used in the production of Chiba tofu. Commercially available powder enzyme preparations, Product A (transglutaminase, manufacturer: Taixing Dongsheng Bio-Tech Co., Ltd., Type TG-TI, Batch No: B20150926, nominal enzyme activity: 116 u/g, actual enzyme activity measured in our laboratory: 108 u/g), and Product B (transglutaminase, manufacturer: Taixing Yiming Biological Co. Ltd., Type TG-B, Batch No: 20150824, nominal enzyme activity: 110u/g, actual enzyme activity measured in our laboratory: 100u/g) were used as controls. The manufacturer of soy protein isolates was Shandong Yuwang Group; Cassava denatured starch was of Rose Brand; soybean oil was provided by COFCO Corporation; I+G and gravy salt were purchased from METRO supermarket; and Control C was a powder enzyme preparation Biobond TG-I produced by Kinry Biotech (Jinan) Co., Ltd., actual enzyme activity measured: 110 u/ml. Chiba tofu was prepared according to the formula and the process as shown in Table 25-1.

**Table 25-1. Use of liquid and powder transglutaminase in the production of Chiba tofu**

| Item | Sample from Example 14 | Control A | Control B | Control C |
|---|---|---|---|---|
| Enzyme dose | 200ml | 200g | 200g | 200g |
| Soy protein | 15kg | 15kg | 15kg | 15kg |
| Cassava denatured starch | 3kg | 3kg | 3kg | 3kg |
| Edible soybean oil | 15kg | 15kg | 15kg | 15kg |
| Edible salt | 200g | 200g | 200g | 200g |
| Gourmet powder | 200g | 200g | 200g | 200g |
| Ice water (1:4) | 70kg | 70kg | 70kg | 70kg |
| Processing procedure | In accordance with the formula, soy protein isolate was added to a 250L chopping pot, ice water was added, and a transglutaminase preparation was added in a specified amount. After high-speed chopping at 2800 rpm/min for 2 min, soy protein was completely swollen. Soybean oil was added, and high-speed chopping was further performed for 2-3 min, and the slurry was completely emulsified and turned white. Modified starch, edible salt and gourmet powder were added, and high-speed chopping was further performed for 1 min. The slurry turned into a milky white, homogeneous emulsified slurry. The emulsified slurry was placed in a 80 cm x 60 cm x 8 cm (length x width x depth) stainless steel tray. The tray was covered with a plastic cloth, and kept in a 50 °C steam box for 1 h, and then was further kept for 1 h in the steam box with the temperature increased to 90 °C. The steel tray was taken out, and the product was cut into slices after air cooling. | | | |

Chiba tofu produced by using different transglutaminase preparations were tested for gel strength, elasticity, color and luster, mouthfeel, etc. The results were shown in Table 25-2.

**Table 25-2. Evaluation on quality of Chiba tofu produced by liquid and powder transglutaminase**

| Test items | Sample from Example 14 | Control A | Control B | Control C |
|---|---|---|---|---|
| Gel strength (g/cm2) | 1036 | 958 | 946 | 963 |
| Elasticity (mm) | 7.188 | 6.771 | 6.282 | 6.845 |
| Color | milky white | milky white | milky white | milky white |
| Mouthfeel | Excellent | Excellent | slightly soft | Excellent |
| Texture | exquisite | exquisite | exquisite | exquisite |
| Flavor | Pure taste and palatable | Pure taste | Pure taste | Pure taste and palatable |
| Resistant to boiling water | Excellent | Excellent | Good | Excellent |
| Change after freezing at -18 °C for 48 h | No visible ice crystal | No visible ice crystal | No visible ice crystal | No visible ice crystal |

The results of experimental test showed that the liquid preparation of transglutaminase was comparable to the powder transglutaminase preparations in the application in Chiba tofu, and the powder preparation could be entirely replaced by the liquid preparation during the production of Chiba tofu. The Chiba tofu thus produced had its quality improved to some extent. The liquid preparation of transglutaminase had commercial value.

The above examples are only some preferred embodiments and application examples of the invention, and the invention are not limited to them.

## Claims

1. A liquid enzyme preparation, **characterized in that** the liquid enzyme preparation is a liquid enzyme preparation of transglutaminase EC2.3.2.13 having an enzyme activity of 10-1000 u/ml **characterized in that** the liquid enzyme preparation has the following physicochemical properties:
1) having a pH of 5.0-9.0;
2) having a water activity A_{w} of ≤O.89; and
3) having a redox potential of -400mv to +50mv.

2. The liquid enzyme preparation according to claim 1, **characterized in that** its components and amounts thereof are as follows: the liquid preparation of transglutaminase has an enzyme activity of 10-1000 u/ml, a water activity regulator is present in an amount of 30-80 w/v%, a redox potential regulator is present in an amount of 0.0075-1 w/v%, a food preservative is present in an amount of 0-0.1 w/v%, and a pH regulator is added to a final volume of 100%.

3. The liquid enzyme preparation according to claim 1, **characterized in that** the liquid enzyme preparation preferably has the following physicochemical properties:
1) having a pH of 5.0-7.5;
2) having a water activity A_{w} of 0.6-0.85; and
3) having a redox potential of -400mv to 0mv.

4. The liquid enzyme preparation according to claim 2, **characterized in that** the water activity regulator is selected from the group comprising of sorbitol, maltitol, propylene glycol, glycerol, xylitol, polyethylene glycol, trehalose, sucrose, maltose, isomaltose, maltodextrin, xylitol, and mannitol.

5. The liquid enzyme preparation according to claim 2, **characterized in that** the redox potential regulator comprises one or more of L-ascorbic acid and a salt thereof, L-serine and a salt thereof, L-cysteine and a salt thereof, reduced glutathione, tea polyphenol, soybean protein hydrolysate, wheat protein hydrolysate, casein hydrolysate, chitosan hydrolysate, bamboo leaf antioxidant, rosemary extract, liquorice antioxidant extract, superoxide dismutase, glucose oxidase, sodium bisulfite, sodium metabisulfite, potassium metabisulfite, sodium sulfite, sodium hyposulfite, phytic acid.

6. The liquid enzyme preparation according to claim 2, **characterized in that** the food additive is selected from the group comprising of ε-polylysine, natamycin, lysozyme, Nisin, potassium sorbate, sodium dehydroacetate, sodium diacetate, methyl p-hydroxybenzoate, ethyl lauroyl arginate HC1, potassium metabisulfite, and sodium metabisulfite.

7. The liquid enzyme preparation according to claim 2, **characterized in that** the pH regulator is one of hydrochloric acid, sulfuric acid, acetic acid, lactic acid, citric acid, malic acid, phytic acid, phosphoric acid, nitric acid, oxalic acid, sodium hydroxide, potassium hydroxide, sodium carbonate, sodium bicarbonate, potassium carbonate, potassium bicarbonate, trisodium citrate, tripotassium citrate, sodium acetate, potassium acetate, sodium lactate, potassium lactate, disodium hydrogen phosphate, dipotassium hydrogen phosphate, sodium phosphate, potassium phosphate, tap water, purified water, and mineral water, or a buffer system consisting of several pH regulators.

8. The liquid enzyme preparation according to claim 2, **characterized in that** the water activity regulator comprises one or more of sorbitol, maltitol, and glycerol; and the pH regulator is a buffer comprising phosphoric acid, acetic acid, lactic acid or citric acid, or a salt thereof.

9. A method for preparing the liquid enzyme preparation according to any one of claims 1-8, **characterized by** the following steps:
1) purification of an enzyme solution: subjecting a crude enzyme solution to pressure filtration, microfiltration, and secondary ultrafiltration, to obtain a purified concentrated enzyme solution;
2) mixing: weighing the purified concentrated enzyme solution, a water activity regulator, and a redox potential regulator quantitatively, adding a food preservative if present, and mixing; adding a pH regulator to a final volume of 100%; after mixing homogeneously, testing and adjusting the liquid enzyme preparation for the following parameters: a pH of 5.0-9.0, a water activity A_{w} of ≤O.89, and a redox potential of -400mv to 50mv;
3) bacteria removal: filtering the resulting mixture through a 0.1-0.22µm membrane for sterilization, followed by sterile filling;
4) package: packaging the liquid enzyme preparation by sterile filling or another relevant liquid package process.

## Patentansprüche

1. Flüssige Enzymformulierung **dadurch gekennzeichnet, dass** die flüssige Enzymformulierung eine flüssige Enzymformulierung von Transglutaminase ist (EC. 2.3.2.13), die eine Enzymaktivität von 10-1000 U/mL besitzt und folgend aufgeführte physiko-chemischen Eigenschaften aufweist
1) einen pH-Wert im Bereich 5.0 bis 9.0
2) eine Wasseraktivität Aw von ≤ 0,89; und
3) ein Redoxpotential zwischen -400 mV und +50 mV

2. Die flüssige Enzymformulierung gemäß Anspruch 1 **dadurch gekennzeichnet, dass** nachfolgend aufgeführte Inhaltsstoffe in genannten Mengen enthalten sind: Die flüssige Enzymformulierung besitzt eine Transglutaminase-Aktivität von 10-1000 U/mL, enthält einen Wasseraktivitätsregulator im Bereich 30-80 % (w/v), einen Redoxpotentialregulator im Bereich 0,0075-1 % (w/v), einen Konservierungsstoff im Bereich 0-0,1 % (w/v) und einen pH-Wert-Regulator in entsprechender Menge, so dass die Gesamtmenge der flüssigen Enzymformulierung 100 % entspricht.

3. Die flüssige Enzymformulierung gemäß Anspruch 1 **dadurch gekennzeichnet, dass** die flüssige Enzymformulierung bevorzugt folgende physiko-chemischen Eigenschaften aufweist:
1) einen pH-Wert zwischen 5,0 und 7,5
2) eine Wasseraktivität (A_{w}) im Bereich von 0,6 bis 0,85; und
3) ein Redoxpotential von -400 mV bis 0 mV

4. Die flüssige Enzymformulierung gemäß Anspruch 2 **dadurch gekennzeichnet, dass** der Wasseraktivitätsregulator ausgewählt wird aus der Gruppe bestehend aus Sorbitol, Maltitol, Propylenglykol, Glycerin, Xylitol, Polyethylenglycol (PEG), Trehalose, Saccharose, Maltose, Isomaltose, Maltodextrin, Xylitol und Mannitol.

5. Die flüssige Enzymformulierung gemäß Anspruch 2 **dadurch gekennzeichnet, dass** der Redoxpotentialregulator beinhaltet einen oder mehrere Stoffe aus der Gruppe bestehend aus L-Ascorbinsäure und einem Salz der Ascorbinsäure, L- Serin und einem Salz von Serin, L-Cystein und einem Salz von Cystein, reduziertem Glutathion, Tee-Polyphenole, Sojaproteinhydrolysat, Weizenproteinhydrolysat, Kaseinhydrolysat, Chitosanhydrolysat, Bambusblattantioxidantien, Rosmarinextrakt, Süßholzantioxidantienextrakt, Superoxiddismutase, Glucoseoxidase, Natriumbisulfit, Natriummetabisulfit, Kaliummetabisulfit, Natriumsulfit, Natriumthiosulfat, Phytinsäure.

6. Die flüssige Enzymformulierung gemäß Anspruch 2 **dadurch gekennzeichnet, dass** der Konservierungsstoff ausgewählt wird aus der Gruppe bestehend aus ε-Polylysin, Natamycin, Lysozym, Nisin, Kaliumsorbat, Natriumdehydroacetat, Natriumdiacetat, Methyl-p- Hydroxybenzoat, EthyllauroylArginate HCl, Kaliummetabisulfit und Natriummetabisulfit.

7. Die flüssige Enzymformulierung gemäß Anspruch 2 **dadurch gekennzeichnet, dass** der pH- Wert-Regulator ausgewählt ist aus der Gruppe bestehend aus Salzsäure, Schwefelsäure, Essigsäure, Milchsäure, Zitronensäure, Äpfelsäure, Phytinsäure, Phosphorsäure, Salpetersäure, Oxalsäure, Natriumhydroxid, Kaliumhydroxid, Natriumcarbonat, Natriumbicarbonat, Kaliumcarbonat, Kaliumbicarbonat, Trinatriumcitrat, Trikaliumcitrat, Natriumacetat, Kaliumacetat, Natriumlactat, Kaliumlactat, Dinatriumhydrogenphosphat, Dikaliumhydrogenphosphat, Natriumphosphat, Kaliumphosphat und Leitungswasser, aufgereinigtem Wasser oder mineralisiertem Wasser oder Puffersystemen basierend auf mehreren pH-Wert-Regulatoren.

8. Die flüssige Enzymformulierung gemäß Anspruch 2 **gekennzeichnet dadurch, dass** der Wasseraktivitätsregulator einen oder mehrere Stoffe enthält ausgewählt aus der Gruppe bestehend aus Sorbitol, Maltitol und Glycerin und der pH-Wert-Regulator ist ein Puffersystem enthaltend Phosphorsäure, Essigsäure, Milchsäure oder Zitronensäure oder ein Salz dieser Säuren.

9. Ein Verfahren zur Herstellung einer flüssigen Enzymformulierung nach einem der Ansprüche 1 bis 8 **gekennzeichnet durch** die folgenden Schritte:
1) Reinigung der Enzymlösung: Behandeln einer nicht aufgereinigten Enzymlösung mit Druckfiltration, Mikrofiltration und weiterer Ultrafiltration, um eine aufgereinigte, konzentrierte Enzymlösung zu erhalten;
2) Mischen: Quantitatives Einwiegen von aufgereinigter, konzentrierter Enzymlösung, Wasseraktivitätsregulator und eines Redoxpotentialregulators sowie ggf. eines Konservierungsstoffes, mischen der Komponenten, Zugabe eines pH-Wert-Regulators bis zu einem finalen Gehalt von 100 %, anschließend homogen mischen, analysieren und Einstellen der Flüssigformulierung auf: einen pH-Wert von 5,0 - 9,0; eine Wasseraktivität A_{w} von ≤ 0,89 sowie ein Redoxpotential von -400 mV bis +50 mV
3) Entkeimung: Sterilfiltration der Lösung mittels einer 0,1-0,22 µm Membran und anschließend Sterilabfüllung
4) Verpackung: Abfüllung der flüssigen Enzymformulierung mittels sterilem Füllen oder einem geeigneten alternativen Prozess zur Flüssigverpackung .

## Revendications

1. Formulation enzymatique liquide **caractérisée en ce que** la formulation enzymatique liquide est une formulation enzymatique liquide de transglutaminase (EC. 2.3.2.13) qui possède une activité enzymatique comprise entre 10 et 1000 U/ml et qui présente les propriétés physico-chimiques mentionnées ci-après
1) un pH compris entre 5,0 et 9,0
2) une activité de l'eau (aw) ≤ à 0,89 ; et
3) un potentiel redox compris entre -400 mV et +50 mV

2. La formulation enzymatique liquide selon la revendication 1 **caractérisée en ce qu'**elle contient les composants mentionnés ci-après dans les quantités indiquées : la formulation enzymatique liquide possède une activité de la transglutaminase comprise entre 10 et 1000 U/ml, elle contient un régulateur de l'activité de l'eau qui se situe dans la plage comprise entre 30 et 80 % (w/v), un régulateur du potentiel redox qui se situe dans la plage comprise entre 0,0075 % et 1 % (w/v), un agent conservateur qui se situe dans la plage comprise entre 0 et 0,1 % et un régulateur de pH en quantité appropriée, de sorte que la quantité totale de la formulation enzymatique liquide correspondant à 100 %.

3. La formulation enzymatique liquide selon la revendication 1 **caractérisée en ce que** la formulation enzymatique liquide présente de préférence les propriétés physico-chimiques suivantes :
1) un pH compris entre 5,0 et 7,5
2) une activité de l'eau (aw) située dans la plage comprise entre 0,6 et 0,85 ; et
3) un potentiel redox compris entre -400 mV et 0 mV

4. La formulation enzymatique liquide selon la revendication 2 **caractérisée en ce que** le régulateur de l'activité de l'eau est choisi dans le groupe constitué par le sorbitol, le maltitol, le propylène glycol, la glycérine, le xylitol, le polyéthylène glycol (PEG), le tréhalose, le saccharose, le maltose, l'isomaltose, la maltodextrine, le xylitol et le mannitol.

5. La formulation enzymatique liquide selon la revendication 2 **caractérisée en ce que** le régulateur de potentiel redox contient une ou plusieurs substances issues du groupe constitué par l'acide L-ascorbique et un sel de l'acide ascorbique, la L-sérine et un sel de la sérine, la L-cystéine et un sel de la cystéine, le glutathion réduit, les polyphénols du thé, l'hydrolysat de protéines de soja, l'hydrolysat de protéines de blé, l'hydrolysat de caséine, l'hydrolysat de chitosane, les antioxydants de feuilles de bambou, l'extrait de romarin, l'extrait d'antioxydants de la réglisse, les superoxydes dismutases, la glucose oxydase, le bisulfite de sodium, le métabisulfite de sodium, le métabisulfite de potassium, le sulfite de sodium, le thiosulfate de sodium, l'acide phytique.

6. La formulation enzymatique liquide selon la revendication 2 **caractérisée en ce que** l'agent conservateur est choisi dans le groupe constitué par la poly-lysine (ε-Potytysin), la natamycine, le lysozyme, la nisine, le sorbate de potassium, le déshydroacétate de sodium, le diacétate de sodium, le 4-hydroxybenzoate de méthyle, l'éthyl lauroyl arginate HCl, le métabisulfite de potassium et le métabisulfite de sodium.

7. La formulation enzymatique liquide selon la revendication 2 **caractérisée en ce que** le régulateur du pH est choisi dans le groupe constitué par l'acide chlorhydrique, l'acide sulfurique, l'acide acétique, l'acide lactique, l'acide citrique, l'acide malique, l'acide phytique, l'acide phosphorique, l'acide nitrique, l'acide oxalique, l'hydroxyde de sodium, l'hydroxyde de potassium, le carbonate de sodium, le bicarbonate de sodium, le carbonate de potassium, le bicarbonate de potassium, le citrate trisodique, le citrate tripotassique, l'acétate de sodium, l'acétate de potassium, le lactate de sodium, le lactate de potassium, l'hydrogénophosphate de sodium, l'hydrogénophosphate de potassium, le phosphate de sodium, le phosphate de potassium, l'eau du robinet, l'eau purifiée ou l'eau minéralisée ou des systèmes tampons basés sur plusieurs régulateurs de pH.

8. La formulation enzymatique liquide selon la revendication 2 **caractérisée en ce que** le régulateur de l'activité de l'eau contient une ou plusieurs substances choisie(s) dans le groupe constitué par le sorbitol, le maltitol et la glycérine, et **en ce que** le régulateur du pH est un système tampon contenant de l'acide phosphorique, de l'acide acétique, de l'acide lactique ou de l'acide citrique, ou un des sels dérivés de ces acides.

9. Un procédé pour la fabrication d'une formulation enzymatique liquide selon l'une des revendications 1 à 8 **caractérisé par** les étapes suivantes :
1) Purification de la solution enzymatique : traitement d'une solution enzymatique non purifiée par filtration sous pression, microfiltration et autre ultrafiltration afin d'obtenir une solution enzymatique purifiée et concentrée ;
2) Mélange : pesage quantitatif de la solution enzymatique purifiée et concentrée, du régulateur de l'activité de l'eau et d'un régulateur du potentiel redox, ainsi que, le cas échéant, d'un agent conservateur, mélange des composants, ajout d'un régulateur de pH jusqu'à obtention d'une teneur finale de 100 % ; mélanger ensuite de manière homogène, analyser et ajuster la formulation liquide à : un pH compris entre 5,0 et 9,0 ; une activité de l'eau (aw) ≤ à 0,89 ainsi qu'un potentiel redox compris entre -400mV et +50mV
3) Désinfection : filtration stérile de la solution au moyen d'une membrane de 0,1 à 0,22 µm, puis remplissage aseptique
4) Conditionnement : remplissage de la formulation enzymatique liquide par voie aseptique ou au moyen d'un procédé alternatif approprié d'emballage des liquides.
